# EUROPEAN PATENT SPECIFICATION

(11) **EP 2 825 186 B1**
(45) Date of publication and mention of the grant of the patent: **18.05.2016**
(21) Application number: 13708428.1
(22) Date of filing: 08.03.2013
(51) Int. Cl.: A61K 36/58, A61K 36/8962, A61P 17/02

(54) **COMPOSITION COMPRISING ALLIUM EXTRACTS AND NEEM OIL**
ZUSAMMENSETZUNG MIT ALLIUMEXTRAKTEN UND NIEMBAUMÖL
COMPOSITION COMPRENANT DES EXTRAITS D'ALLIUM ET DE L'HUILE DE MARGOUSIER

(30) Priority: 12.03.2012 IT BO20120123
(43) Date of publication of application: 21.01.2015
(73) Proprietor: Ri.Mos. S.r.l., 41037 Mirandola (MO) (IT)
(72) Inventor: CASELLA, Sergio, I-44100 Lucca (IT)
(74) Representative: Negrini, Elena
(86) International application number: PCT/EP2013/054798
(87) International publication number: WO 2013/135594

(56) References cited:
- WO-A1-2008/097109
- WO-A2-2006/013607
- SUNDAY E ATAWODI ET AL: "Azadirachta indica (neem): a plant of multiple biological and pharmacological activities", PHYTOCHEMISTRY REVIEWS, KLUWER ACADEMIC PUBLISHERS, DO, vol. 8, no. 3, 3 September 2009 (2009-09-03), pages 601-620, XP019752732, ISSN: 1572-980X, DOI: 10.1007/S11101-009-9144-6
- SOUAD AKROUM ET AL: "Antimicrobial, antioxidant, cytotoxic activities and phytochemical screening of some Algerian plants", EUROPEAN JOURNAL OF SCIENTIFIC RESEARCH, EUROJOURNALS PUBLISHING, GB; IT, vol. 31, no. 2, 1 January 2009 (2009-01-01), pages 289-295, XP002645199, ISSN: 1450-216X
- WIEST J M ET AL: "Inibicao e inativacao de Escherichia coli por extratos de plantas com indicativo etnogràfico medicinal ou condimentar // Escherichia coli inhibition and inactivation by extracts from plants with medicinal and spice ethnographic indicative", CIENCIA E TECNOLOGIA DE ALIMENTOS, SOCIEDADE BRASILEIRA DE CIENCIA E TECNOLOGIA DE ALIMENTOS, CAMPINAS, BR, vol. 29, no. 3, 1 July 2009 (2009-07-01), pages 474-480, XP002645200, ISSN: 0101-2061

## Description

### Technical Field

The present invention relates to a composition containing extract derived from Allium genus, in particular Allium Porrum L., and Neem oil having anti-microbial, anti-bacterial, anti-septic, anti-fungal, repellent, healing and tissue regenerating activity.

### Background Art

The use of plant derivatives as therapeutic agents is known for thousands of years. In particular, the therapeutic uses of extracts or substances derived from Allium genus plants, particularly cepa and sativum are known in literature; for example are described as systemic anti-bacterial, anti-fungal, platelet anti-aggregate and diuretic activities, used as diabetes treatment, for atherosclerosis prevention, anti-hypertensive, antiasthmatic and for the bronchitis treatment and the Neem oil is also well known for its anti-inflammatory, anti-septic and healing activity.

### Disclosure of Invention

Object of the present invention is a composition comprising an extract derived from Allium Porrum L. genus plants, in vegetable oil and Neem oil.

The composition object of the present invention may optionally comprise an additional oil or composition of oils having therapeutic activity.

The composition object of the present invention has healing, tissue regeneration and anti-microbial properties that make it particularly effective for the treatment of injuries, inflammations and infections of the human body and animal both acute and chronic.

Object of the present invention is a composition (and the use thereof) which comprises a composition for the treatment of injuries, inflammations and infections both acute and chronic of both human and animal body.

This invention is based on a composition or mix comprising an extract in vegetable oil of Allium Porrum L. genus plants and Neem oil, and optionally also other vegetable and/or essential oils, with effectiveness far superior with respect to the sum of each component effectiveness.

The vegetable oil to obtain the extract of Allium Porrum L. (also called Leek) may be the same Neem oil thus obtaining a base composition consisting of only two vegetable origin elements to which may optionally be added other oils.

This invention is therefore based on the combination of Allium Porrum L. plant extract and Neem oil, and optionally with one or more additional vegetable oils or with one or more essential oils or their compositions having anti-bacterial, anti-fungal, anti-viral, anti-septic, repellents, healing properties and direct anti-microbial activity (anti-bacterial, anti-fungal, anti-viral) otherwise absent in the Allium Porrum L. plant extract alone and not obtainable, at least in equal amounts, by means of the other constituents, thereby making the association particularly effective in vivo for the external and internal both acute and chronic lesions treatment of the human and animal body.

The healing and tissue regeneration and direct anti-microbial properties (anti-bacterial, anti-fungal, anti-viral) in vivo is not known relating to an association of Allium Porrum L. extract and Neem oil and as nowadays there isn't any known composition of plant extracts having the same therapeutic activity.

Therefore it is object of the present invention a composition which comprises an extract derived from Allium Porrum L. genus plants, and Neem oil with optional addition of vegetable oil.

The amount of extract derived from Allium Porrum genus plants in vegetable oil may be comprise in the composition in a range between 1% and 99% by weight, preferably between 5% and 70%.

The amount of Neem oil can be comprised, in the composition, in a range between 1% and 99% by weight, preferably between 30% and 95%.

The composition object of the present invention may optionally comprise an additional oil or composition of oils having therapeutic activity.

Examples of vegetable oils that can be used in the compositions according to the present invention to obtain the Allium Porrum L. extract or as optional ingredients or constituents are: olive oil, seed oil, sweet almond oil, jojoba oil or preferably extra virgin olive oil.

Examples of essential oils having anti-microbial activity that can be used in the compositions according to the present invention are: essential oil of Artemisia dracunculus, Carum carvi, Cedrus atlantica, Cinnamomun verum, Citrus aurantium, Citrus bergamia, Coriadrum sativum, Cupressus sempervirens, Eucalyptus citriodora, Eucalyptus dives, Eucaliptus globulus, Eucalyptus radiata, Eucarya spicata, Foeniculum vulgare, Hyssopus officinalis, Lavandula angustifolia, Melaleuca alternifolia, Melaleuca leucadendron, Melaleuca viridiflora, Mentha piperita, Myristica fragrans, Ocimum basilicum, Origanum majorana, Ormenis mixta, asperum Pelargonium (geranium), Pimpinella anisum, Pinus sylvestris, Piper nigrum, Rosa damascena, Rosmarinus officinalis, Salvia officinalis, Satureja ortensis, Satureja montana, Syzygium aromaticum, Thymus capitatum, Thymus serpillum, Thymus mastichina, Thymus vulgaris ct. timol, ct. carvacrolo, ct. linalolo, ct. geraniolo, Chamomilla recutita, Coriandrum sativum, Cuminum cyminum, Nardostachys jatamansi, Pelagronium graveolens, Tagetes glandulifera, Citrus limon, Commiphora molmol, Lavandula latifolia, Melissa officinalis, Ravensara aromatic, etc..

Thymus Serpillum essential oil is particularly preferred.

When present, the essential oil amount in the compositions object of the present invention is up to a maximum of 15% by weight, preferably ranging between 1 % and 8%.

The composition object of the present invention has many advantages with respect to what is currently in use for lesions treatment as it makes unnecessary the disinfectants and antibiotics use.

The composition of the invention is used as a medicament in the form of a composition/solution/maceration composed from a cold or hot extract of Allium Porrum L. bulbs in vegetable oil, and Neem oil and any other vegetable origin and/or essential oils.

The composition/solution is prepared by mixing (a) the Allium Porrum L. extract in vegetable oil, (b) the Neem oil and (c) any other optional essential oil at different concentrations all included in the values shown in the following Table 1 in such a way that the sum of (a) + (b) + (c) is equal to 100.

**Table 1 - Component concentration (%)**

| (a) | (b) | (c) |
|---|---|---|
| From 1 to 99 | From 1 to 99 | From 0 to 15 |

The composition/solution is filtered providing the composition object of the invention.

In the maceration extraction case the extraction is realized directly extracting the vegetable in the Neem oil and/or other vegetable oils.

The composition is coloured depending on the used oils, has characteristic smell, long time lasting, does not go rancid and needs no preservatives. It can be used directly as an ointment without the addition of other ingredients or can be added with beeswax obtaining a denser ointment.

For use as a medicament in other forms, gel and/or emulsions can be added as lipophilic component, and the composition can be also electro wired in order to provide a particular medicament form know as "scaffold" that can be resorbable or non-resorbable or it can be adsorbed on medicated gauzes. It can also be ozonated to increase therapeutic speed rate.

Other suitable medicament forms are toothpaste, mouthwash, aqueous solutions, tablets, capsules and phials.

The medicaments prepared using the composition object of the present invention are particularly effective due to their anti-inflammatory, anti-microbial, anti-bacterial, anti-septic and repellents, anti-fungal, anti-viral healing and cellular tissue regeneration properties.

They are therefore particularly useful for healing external and/or internal lesions of human or animal body, supported by microbes, viruses, bacteria and fungal infections, and lesions supported by circulatory problems, including sores. They are also useful on cutaneous inflammatory phenomena treatment such as atopic dermatitis both on animal and human.

The compositions of the invention can therefore be used as a suitable medicament for treating inflammation, orifices infection in humans or animals, such as the oral, auditory, anal and vaginal cavities and for treating intestinal and stomach ulcers.

### Preparation examples of the composition

Two preparation examples of the composition are in the followed explained.

### Example 1

Providing an amount of fresh Allium Porrum and cutting leaves and roots: preferably using the bulbs.

Grinding the bulbs into small pieces.

Inserting oily matrix in a closed vessel, heat-resistant up to 100°C, equipped with motorized blades.

In this case, the extraction is achieved in Neem oil (oily matrix).

Pre-heating the Neem oil up to a temperature ranging between 70÷85°C, rotating the blades on a range between 30 and 50rpm.

Putting the Allium Porrum pieces in the oily matrix within the vessel and processing for a time ranging between 50 and 70min.

After the extraction process, solid part is subjected to pressing: the resulting liquid will be added to the oily matrix and everything will be filtered: 1 to 50µm.

After cooling down the liquid in a natural way and it is ready for use or further processing for further packaging.

### Example 2

The Allium Porrum preparation is the same of Example 1.

In a blade equipped vessel putting an oily matrix consisting of extra virgin olive oil.

Pre-heating said oily matrix up to 70÷75°C rotating the blades at a speed ranging between 30÷50rpm.

At this point, inserting the shredded Allium Porrum and bringing it up to 90÷105°C.

Processing for a time ranging between 30÷45min.

After the extraction and after the pressing of the solid residue all the remaining oil is filtered.

Cooling down the mass at about 50°C and adding an equal amount of Neem oil.

At the end adding the Thymus Serpillum essential oil, in an amount such as to obtain the 5% of the total liquid mass.

The liquid is then cooled down at room temperature and is ready for use or for further processing to obtain other forms such as gel scaffold, pastries, etc.

### Effectiveness examples

The obtained composition according to this method is characterized by a rapid and potent healing activity and a remarkable anti-bacterial activity also in unhealthy environments.

### Example 3

Mare with over-orbital lesion of traumatic origin due to fence impact.

When the treatment has been started, the lesion has been present by 10 days and there has been also a significant infection.

Three days treatment with liquid form product, by means of mechanical pump spray container (to arrive in the depth of the lesion), has been sufficient to achieve the infection disappearance after the first day and the complete recovery at the end of the indicated period.

### Example 4

Cinta Senese sow.

Bedsore as a result of prolonged immobility due to postpartum breakdown.

Chronic lesion with bacterial infection presence.

Treatment with gel form product to ensure a longer contact time and a protection towards the unhealthy environment (defecations, etc.).

Treatment duration: 20 days to obtain a total recovery.

### Trial and experimental results

The trial results are in the followed evidenced with particular reference to the images and graphics, wherein:

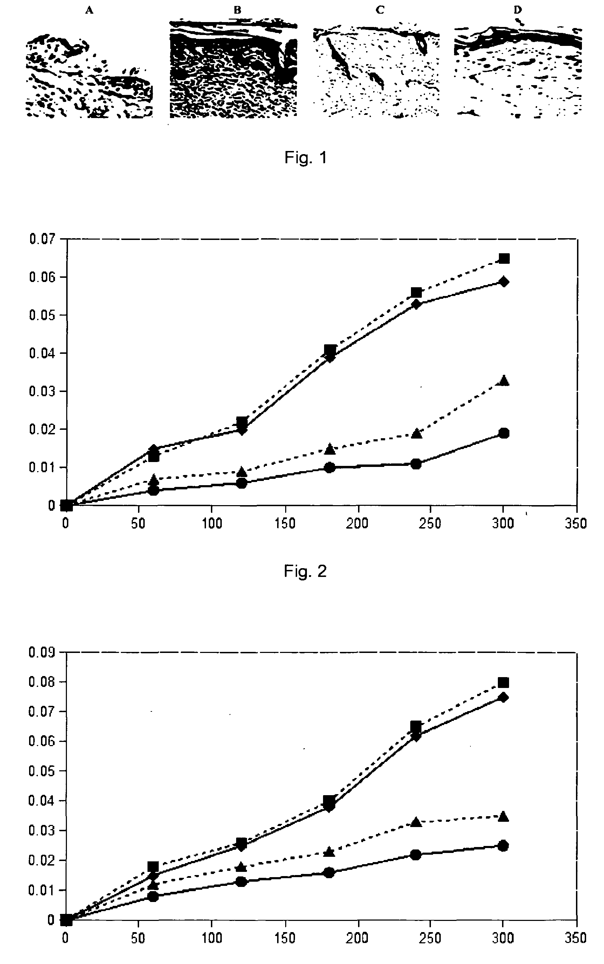

Figures 1A-1D show respectively:
   - a scar/injured, untreated tissue having irregular re-epithelialisation, sparse fibroblasts and minimal collagen deposition;
   - an injured tissue treated by means of pure Neem oil with fibroblasts repopulation and collagen deposition;
   - an injured tissue treated by means of pure Leek extract having thin, continuous epithelial layer;
   - an injured tissue treated by means of synergistic composition of Neem oil and Leek extract having regular re-epithelialisation, regular collagen deposition and no keloid tissue evidence.

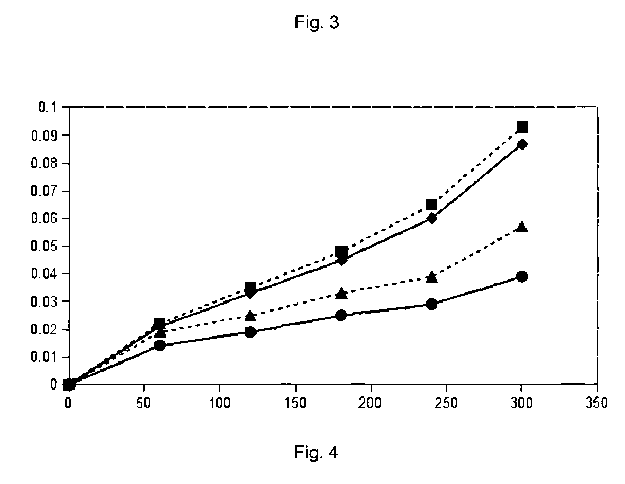

Figures 2 to 4 shows several graphs of bacterial growth evaluated by means of OD600nm spectrophotometry versus time (minutes) wherein the dotted line with square marks relates to untreated tissue; the dotted line with triangle marks relates to pure Neem oil treated tissue; the line with rhombus marks relates to pure Leek extract treated tissue; the line with circle marks relates to tissue treated by means of the composition of Neem oil and Leek extract; and in detail:
   - Figure 2 shows E. Coli bacterial growth;
   - Figure 3 shows P. Aeruginosa bacterial growth;
   - Figure 4 shows S. Aureus bacterial growth.

As a first step has been examined the effect epithelising of the composition object of the present invention.

### Lesion reduction speed rate evaluation

The reduction lesion area speed rate evaluation has been conducted on the basis of the following method: four patients groups selected with reference to comparable surface and depth of initial lesion area have been monitored through clinical observation to evaluate the recovery process evolution.

19 patients have been enlisted. The control group (treated by means of sterile physiological salt solution) consists of four subjects whose initial lesion area is smaller than the other groups; the choice not to subject the patient to any type of treatment could in fact only be performed on patients with limited lesion areas; this data is then compared with the other groups but not for absolute regression percentage.

The patients were heterogeneous about age (average age 64.3 ± 7.3 years old) and sex; the considered spontaneous and random lesions were acute (burns, lacerations, and/or skin lesions due to small trauma), and related almost exclusively to the lower limbs. The composition of Neem oil and Leek extract were applied twice a day for a period of 28 days, significant for the desired evaluation. In no case has been found allergic reactions occurrence or general or topical clinical intolerance. During the clinical observation the medicating change has been performed without pain and without obvious alteration signs of the near lesion area or the ulcered bottom. The lesion area evaluation has been performed with the method of track-grider on triacetate sheet. The precision of the method has been maximized through the use of a camera connected to the computer monitor that has allowed an accurate and reproducible evaluation on behalf of at least two independent operators.

Table 2: reduction effect of the recovery time of the lesion area after single treatment and in association (composition of the invention comprising Allium Porrum extract and Neem oil) with respect to the control group.

**Table 2 - Lesion area (cm2)**

| | Experimental group | | | |
|---|---|---|---|---|
| Time (days) | Control (untreated) (n*=4) | Pure Leek treatment (n=5) | Pure Neem treatment (n=5) | Combination treatment (n=5) |
| 0 | 3.35 ± 0.05 | 8.25 ± 0.12 | 7.40 ± 0.11 | 7.95 ± 0.40 |
| 3 | 2.95 ± 0.20 | 5.15 ± 0.31 | 6.90 ± 0.70 | 4.10 ± 0.05 |
| 7 | 2.55 ± 0.09 | 3.95 ± 0.29 | 4.10 ± 0.11 | 3.80 ± 0.22 |
| 14 | 2.05 ± 0.02 | 2.60 ± 0.05 | 2.65 ± 0.15 | 1.90 ± 0.05 |
| 21 | 1.85 ± 0.04 | 1.45 ± 0.21 | 2.05 ± 0.31 | 0.75 ± 0.01 |
| 28 | 1.55 ± 0.03 | 0.75 ± 0.02 | 1.30 ± 0.04 | 0.70 ± 0.01 |

| | | | | |
|---|---|---|---|---|
| n* indicates the number of subjects included in the trial belonging to respective group. | | | | |

### Results

After the third day of the experimental period, the treatment with the composition of Leek extracts and Neem oil, object of the present invention, showed a significant reduction in the lesion area compared to single component treatments and the control group. Analyzing the temporal recovery evolution is possible to note that the treatment leads to a lesion reduction in accelerated time. In particular, it can be seen how the lesion area may be reduced by over 90% (90.56%) after only 21 days from the treatment beginning; this result is highly significant to a considerable synergistic effect whereas the individual treatments lead to a lesion reduction of 82.43% (pure Neem oil) and 90.90% (pure Leek) in a time of 28 days (while at the time of 21 days the reduction has been respectively 82% for pure Leek and of 72,29% for pure Neem).

The obtained results indicate that by the treatment leads to a speeding up in the lesion area reduction of at least 25% over 28 days of follow-up and observation.

### Histological analysis of lesion tissues and their regeneration

A histological analysis of lesion tissues and their regeneration has been conducted (4 days post lesion Table 2).

Referring to Figure 1A-1D where there are illustrated:
- a scar/injured, untreated tissue having irregular re-epithelialisation, sparse fibroblasts and minimal collagen deposition;
- an injured tissue treated by means of pure Neem oil with fibroblasts repopulation and collagen deposition;
- an injured tissue treated by means of pure Leek extract having thin, continuous epithelial layer;
- an injured tissue treated by means of synergistic composition of Neem oil and Leek extract having regular re-epithelialisation, regular collagen deposition and no keloid tissue evidence;
there are the following results: Treatment with the composition object of the present invention allows stimulating the natural repair of lesion tissue in a faster and complete than the individual treatments and the natural course. In this recovering process is evident the re-epithelialisation of the tissue that allows to obtain aesthetic and functional results comparable to the pre-lesion, without result in scar (or keloid) or white atrophy (tissue connector repairing).

### Evaluation of in vitro bacterial growth effect

It has been carried out the evaluation of in vitro bacterial growth effect under the influence of Neem oil and Leek extract (pure or in combination as required in the composition object of the present invention) about bacterial growth of E. Coli, S. Aureus and P. Aeruginosa.

Method: the pre-bacterial cultures were cured at 37°C for 12 hours to obtain the experiment starting bacterial population. The inoculum has been then seeded at concentration (but identical for each sample) of about 1.000.000/ml in LB broth wherein it has been previously solubilised the amount of oil/vegetable extract determined through preliminary experiments, carried out at Leek/Neem concentrations ranging between 25-75% by weight. The 600nm readings have been taken every 60min by means of a Thermo Scientific spectrophotometer. The bacterial blocks selected for the experiment were Escherichia Coli, Pseudomonas Aeruginosa, Staphylococcus Aureus as one of the main causes of nosocomial of also skin infections.

Referring to , "E. Coli bacterial growth evaluated by means of OD600nm spectrophotometry", it has been observed the following result: the composition of the present invention, comprising the association of Neem oil with Leek extract, involves a considerable reduction in the potential E. Coli bacterial growth compared to the individual treatments and the untreated control sample. In particular, the inhibition growth effect appears to be more evident in using pure Neem oil with respect to the Leek extract (on an average Neem oil inhibits the growth of at least 30-50% with respect to control and Leek extract which are almost comparable for the purposes of bacterial growth). The combination of the two active principles leads to a bacterial growth reduction ranging between 38-43% compared to pure Neem oil suggesting that the use of the composition of Neem oil and Leek Extract in skin lesion treatment (and generally in E. Coli infection risk situations) can results in a valid tool for controlling the bacterial infection.

Referring to , "P. Aeruginosa bacterial growth evaluated by means of OD600nm spectrophotometry", it has been the following result: the composition object of the present invention involves a considerable reduction in the P. Aeruginosa bacterial growth curves. In particular it is possible to note that at 240min, the treatment synergy causes a reduction of the bacterial growth equal to 33.33% with respect of treatment with pure Neem oil and to 65% with respect of treatment with pure Leek extract (showing individually a negligible ability in bacterial growth reducing). This result indicates that treatment with the composition of the invention involves a considerable advantage in terms of P. Aeruginosa bacterial growth inhibition.

Referring to , "S. Aureus bacterial growth evaluated by means of OD600nm spectrophotometry", it has been observed the following result: the composition of the invention, comprising the association of Neem oil with Leek extract, involves an important reduction in S. Aureus potential bacterial growth. In particular, the growth inhibition effect appears to be maximized by the synergistic association at 300min when the combination of the oils is able to reduce bacterial growth of a significant value (in particular, there has been a reduction of 31, 57% compared to pure Neem oil and of 55.17% compared to pure Leek extract and of 58.06% compared to control). This result shows how the composition of Neem oil and Leek extract creates an extremely unfavourable environment to S. Aureus bacterial growth, which is significantly inhibited.

## Claims

1. Composition **characterized in** comprising a composition of extracts from Allium Porrum L. genus plant, in vegetable oil and Neem oil.

2. Composition according to claim 1 further containing a vegetable or essential oil having therapeutic activity or compositions thereof.

3. Composition according to claim 1 wherein the amount of extract derived from a plant of the Allium genus in vegetable oil is between 1 % and 99% of weight.

4. Composition according to claim 1 wherein the amount of Neem oil is between 1 % and 99% of weight.

5. Composition according to claim 1 wherein the vegetable oil of Allium Porrum L. extract, is chosen among olive oil, extra virgin olive oil, seed oil, sweet almond oil, jojoba oil, Neem oil .

6. Composition according to claim 5 wherein the vegetable oil is extra virgin olive oil.

7. Composition according to claim 2 containing an essential oil.

8. Composition according to claim 7 wherein the amount of essential oil is until a maximum of 15%.

9. Composition according to claim 1 for use in treating of wounds, inflammations and infections of the human and animal body both acute and chronic, both internal and external. .

10. Composition for use according to claim 9 wherein it is an unguent, a gel, an emulsion, a scaffold, a tooth-paste, a mouthwash, an aqueous solution, pills, capsules or phials.

## Patentansprüche

1. Zusammensetzung, **dadurch gekennzeichnet, dass** sie aufweist eine Zusammensetzung aus Extrakten der Allium Porrum L. Pflanzengattung in Pflanzenöl und Neemöl.

2. Zusammensetzung gemäß Patentanspruch 1, weiterhin enthaltend ein pflanzliches oder ätherisches Öl mit therapeutischer Aktivität oder Zusammensetzungen davon.

3. Zusammensetzung gemäß Patentanspruch 1, wobei die Menge an Extrakt, die aus einer Pflanze der Allium-Gattung erhalten wird, in Pflanzenölen zwischen 1 Gew.-% und 99 Gew.-% liegt.

4. Zusammensetzung gemäß Patentanspruch 1, wobei die Menge an Neemöl zwischen 1 Gew.-% und 99 Gew.-% liegt.

5. Zusammensetzung gemäß Patentanspruch 1, wobei das pflanzliche Öl aus Allium Porrum L. Extrakt ausgewählt ist aus Olivenöl, extranativem Olivenöl, Samenöl, süßem Mandelöl, Jojobaöl, Neemöl.

6. Zusammensetzung gemäß Patentanspruch 5, wobei das Pflanzenöl ein extranatives Olivenöl ist.

7. Zusammensetzung gemäß Patentanspruch 2, enthaltend ein ätherisches Öl.

8. Zusammensetzung gemäß Patentanspruch 7, wobei die Menge an ätherischem Öl maximal 15% beträgt.

9. Zusammensetzung gemäß Patentanspruch 1 zur Verwendung bei der Behandlung von Wunden, Entzündungen und Infektionen des menschlichen und tierischen Körpers, sowohl akute als auch chronische sowie innerliche und äußerliche.

10. Zusammensetzung zur Verwendung gemäß Patentanspruch 9, in der Darreichungsform einer Salbe, eines Gels, einer Emulsion, eines Gerüsts/Trägers, einer Zahnpasta, eines Mundwassers, einer wässrigen Lösung, von Pillen, von Kapseln oder von Ampullen.

## Revendications

1. Composition **caractérisée en ce qu'**elle comprend une composition d'extraits de plante du genre Allium Porrum L., dans de l'huile végétale et de l'huile de margousier.

2. Composition selon la revendication 1, contenant de plus une huile végétale ou essentielle ayant une activité thérapeutique ou des compositions de celle-ci.

3. Composition selon la revendication 1, dans laquelle la quantité d'extrait dérivé d'une plante du genre Allium dans de l'huile végétale se situe entre 1 et 99 % en poids.

4. Composition selon la revendication 1, dans laquelle la quantité d'huile de margousier se situe entre 1 et 99 % en poids.

5. Composition selon la revendication 1, dans laquelle l'huile végétale de l'extrait d'Allium Porrum L. est choisie parmi l'huile d'olive, l'huile d'olive extra vierge, l'huile de graines, l'huile d'amande douce, l'huile de jojoba, l'huile de margousier.

6. Composition selon la revendication 5, dans laquelle l'huile végétale est de l'huile d'olive extra vierge.

7. Composition selon la revendication 2 contenant une huile essentielle.

8. Composition selon la revendication 7, dans laquelle la quantité d'huile essentielle est jusqu'à un maximum de 15 %.

9. Composition selon la revendication 1, pour une utilisation dans le traitement de lésions, d'inflammations et d'infections du corps humain et animal à la fois aiguës et chroniques, à la fois internes et externes.

10. Composition pour une utilisation selon la revendication 9, dans laquelle elle est une pommade, un gel, une émulsion, un échafaudage, une pâte de dentifrice, un bain de bouche, une solution aqueuse, des pilules, des capsules ou des flacons.
